# EUROPÄISCHE PATENTSCHRIFT

(11) **EP 1 186 909 B1**
(45) Veröffentlichungstag und Bekanntmachung des Hinweises auf die Patenterteilung: **02.01.2013**
(21) Anmeldenummer: 01115986.0
(22) Anmeldetag: 30.06.2001
(51) Int. Cl.: G01T 1/00, G01T 1/20, A61B 6/03

(54) **Verfahren zur Ermittlung der Materialart**
Method for determining the type of materials
Procédé pour déterminer le type de matériau

(30) Priorität: 07.09.2000 DE 10044357
(43) Veröffentlichungstag der Anmeldung: 13.03.2002
(73) Patentinhaber: Smiths Heimann GmbH, 65205 Wiesbaden (DE)
(72) Erfinder: Frank, Andreas, Dr., 36304 Alsfeld-Leusel (DE); Schall, Patricia, 64331 Weiterstadt (DE); Geus, Georg, 65201 Wiesbaden (DE)
(74) Vertreter: Dietrich, Barbara

(56) Entgegenhaltungen:
- EP-A2- 0 235 387
- EP-A2- 0 467 044
- US-A- 4 963 746
- US-A- 5 831 269
- SYSOEVA E V ET AL: "The study of alpha/gamma ratio for inorganic scintillation detectors" NUCLEAR INSTRUMENTS & METHODS IN PHYSICS RESEARCH, SECTION - A: ACCELERATORS, SPECTROMETERS, DETECTORS AND ASSOCIATED EQUIPMENT, ELSEVIER, AMSTERDAM, NL, Bd. 414, Nr. 2-3, 11. September 1998 (1998-09-11), Seiten 274-278, XP004139169 ISSN: 0168-9002
- ATROSHCHENKO L V ET AL: "Structure defects and phase transition in tellurium-doped ZnSe crystals" JOURNAL OF CRYSTAL GROWTH, ELSEVIER, AMSTERDAM, NL, Bd. 197, Nr. 3, 15. Februar 1999 (1999-02-15), Seiten 475-479, XP004156403 ISSN: 0022-0248

## Beschreibung

Die Erfindung betrifft ein Verfahren zur Zerstörumgsfrein Material prifung, zur Lebensmittelprüfung des zur Kontrolle fon gepäck und Frachtgut durch Ermittlung der Materialart der Gegenstände eines zu durchleuchtenden Objektes.

Zur Bestimmung der Materialart eines durchleuchteten Objektes mittels Röntgenstrahlen sind Detektoranordnungen bekannt, die aus mehreren vorrangig zu Paaren angeordneten Detektoren bestehen. Jedes Detektorpaar besteht dabei aus zwei hintereinander angeordneten Strahlungsdetektoren, die nacheinander von den Röntgenquanten einer Strahlungsquelle durchdrungen werden, wobei der vordere Detektor insbesondere bei den höheren Energien eine geringere Absorption besitzt als der hintere. Röntgenquanten niedriger Energie werden fast vollständig im vorderen Niederenergiedetektor absorbiert. Röntgenquanten höherer Energie passieren den Niederenergiedetektor fast ohne Wechselwirkung und werden im hinteren Hochenergiedetektor absorbiert. Dabei kann der vordere Detektor eine geringere Dicke als der hintere Detektor aufweisen. Zusätzlich können die beiden Detektoren auch unterschiedliche chemische Zusammensetzungen und Dichten besitzen.
Mit einer solchen Anordnung erreicht man eine Auftrennung der Röntgenstrahlung in einzelne Energiebereiche, wodurch eine Bestimmung der Materialarten der Gegenstände des durchleuchteten Objektes möglich wird. Die Strahlungsdetektoren bestehen beispielsweise aus Festkörperszintillatoren in Verbindung mit Halbleiterphotodioden. Diese Festkörperleuchtstoffe (Festkörperszintillatoren) wandeln die Röntgenstrahlung z. B. in sichtbare Strahlung um, die dann durch die Photodioden in ein Stromsignal umgesetzt wird. Die Größe des Stromsignals ist dabei proportional zu der Intensität der absorbierten Röntgenstrahlung.

Eine derartige Anordnung zur Detektion von Röntgenstrahlung ist in US 4,626,688 offenbart. Hierbei werden die einzelnen Energiebereiche der Röntgenstrahlung zeitgleich auf ein zu durchleuchtendes Objekt mit Gegenständen abgestrahlt und dabei abgeschwächt. Als Detektormaterialien werden hierbei polykristalline Phosphore vorgeschlagen, die für niederenergetische Detektoren aus Elementen mit Ordnungszahlen im Bereich von 39 bis 57 und für hochenergetische Detektoren aus Elementen mit Ordnungszahlen im Bereich von 56 bis 83 bestehen. In Abhängigkeit dieser Ordnungszahlen wird die Dicke der einzelnen Detektoren bestimmt, bei der eine gute Szintillation gewährleistet wird. Zwischen den beiden Detektoren ist ein Filtermaterial eingebracht, um somit eine bessere Trennung der beiden Energiebereiche zu bewirken. Nachteilig bei dieser Anordnung ist, daß die Phosphore mit homogener Massenbelegung im Vergleich zu bekannten Einkristallen mit einer homogenen Dicke nur sehr schwer herzustellen sind. Weiterhin besitzen Phosphore gegenüber Einkristallen oft einen verminderten Wirkungsgrad bezüglich der Umwandlung von Röntgenstrahlung in Licht. Dies liegt darin begründet, daß ein Teil des erzeugten Szintillationslichtes innerhalb der Phosphorschicht an den Phosphorpartikeln gestreut und / oder absorbiert wird. Als Folge davon liefern Phosphorschichten ein Signal, das mit einem kleineren Signal-zu-Rauschverhältnis behaftet ist, als das Signal vieler Einkristallszintillatoren. Ein weiterer Nachteil ergibt sich aus der Tatsache, daß bei Detektormaterialien mit einer Ordnungszahl von ungefähr 50 eine K-Absorptionskante des entsprechenden Elements im Bereich von 30 keV und darüber liegt. Enthält der niederenergetische Detektor ein Material eines solchen Elementes, nimmt die unerwünschte Wechselwirkung hochenergetischer Röntgenquanten mit Energien über 50 keV im Niederenergiedetektor zu, wobei der Schwerpunkt des im Niederenergiedetektor absorbierten Röntgenspektrums zum höheren Röntgenspektrum hin verschoben wird, wodurch eine Verschlechterung der Trennung der beiden Energiebereiche bewirkt wird.

Aus der DE 44 02 258 A1 ist ein Detektor zum Nachweis hochenergetischer Strahlungen bekannt, der aus heißgepreßtem Leuchtstoff und einer Photodiode bzw. einem Photomultiplier besteht. Dieser Leuchtstoff, der auch unter dem Begriff gepreßte Keramik bekannt ist, weist gute Szintillationseigenschaften auf. Die hochenergetische Röntgenstrahlung wird im Leuchtstoff absorbiert, wobei als Folge der Absorption sichtbares Licht emittiert und von der Photodiode (photosensitives Element) detektiert wird. Der hierbei offenbarte Leuchtstoff basiert auf Elementen des Selten-Erden-Oxysulfids und weist ein geringes Nachleuchten auf. Auch die DE 44 27 021 A 1 offenbart einen derartigen hochenergetische Strahlen detektierenden Detektor, dessen Detektormaterial zusätzliche Dotierungen aufweist. Verwendung finden derartige Detektoren in der Röntgencomputertomographie

Das US-Patent 4, 963, 746 beschreibt ein Verfahren und Vorrichtung zur Detektion von Röntgenstrahlen verschiedener Energien, um medizinisch diagnostische Bilder zu erzeugen. Ein Niederenergiedetektor ist vor einem Hochenergiedetektor angeordnet, wobei der Niederenergiedetektor aus Elementen der Ordnungszahlen 29 bis 48 besteht, beispielsweise aus YOS. Der Hochenergiedetektor besteht aus Elementen der Ordnungszahlen 61 bis 68, beispielsweise aus G)S oder CdWo₄. Sowohl die Materialien der Niederenergiedetektoren, als auch die Materialien der Hochenergiedetektoren liegen als Phosphore vor.

Aufgabe der Erfindung besteht darin, ein Verfahren anzugeben, mit dessen Hilfe eine bessere Trennung von niederenergetischen und hochenergetischen Energieanteilen eines polychromatischen Röntgenstrahls ermöglicht wird und infolge dessen eine bessere Materialbestimmung des durchleuchteten Objekts geschaffen wird.

Gelöst wird die Aufgabe durch die Merkmale des Patentanspruches 1.

Der Erfindung liegt die Idee zugrunde, durch eine aufeinander abgestimmte Wahl der Materialien eines Niederenergiedetektors und eines Hochenergiedetektors eine bessere Energieauftrennung zu erhalten. Beim erfindungsgemäßen Niederenergiedetektormaterial ist ein Material vorgesehen, das neben einem hohen Wirkungsgrad bei der Umsetzung von Röntgenstrahlung in Licht, eine geringe Selbstabsorption des erzeugten Szintillatorlichts sowie ein an die spektrale Empfindlichkeit der Photodiode angepaßtes Spektrum des Szintillationslichts sowie eine vorteilhafte chemische Zusammensetzung besitzt. Dazu weist das Detektormaterial eines Niederenergiedetektors Elemente im Ordnungszahlbereich von 30 bis 40 auf.
Die Dicke des Niederenergiedetektors wird so gewählt, daß die niederenergetischen Röntgenquanten fast vollständig absorbiert werden, hochenergetische Röntgenquanten mit Energien von beispielsweise über 50 keV jedoch größtenteils den Niederenergiedetektor durchdringen. Durch eine Oberflächenbearbeitung wie z. B. Polieren oder Läppen ist die benötigte hohe Dickenhomogenität erreichbar. Vorteilhafte Ausführungen dieser Niederenergiedetektoren sind gegenüber Feuchtigkeit und Temperaturschwankungen resistent. Diese vorgenannten Eigenschaften weist insbesondere Zinkselenid auf, welches mit Tellur dotiert ist.

Der Hochenergiedetektor besteht vorzugsweise aus einem dichten Material bestehend aus Elementen mit großen Ordnungszahlen im Bereich von 56 bis 83, um diesen Detektor möglichst dünn gestalten zu können. Vorzugsweise ist dieses Detektormaterial ein keramisches Gadoliniumoxylsulfid dotiert mit mindestens einem Selten-Erden-Element SE.

Aufgrund der aufeinander abgestimmten Wahl der Materialien des Niederenergiedetektors und des Hochenergiedetektors weist die Detektoranordnung eine geringe Nachleuchtdauer und damit eine deutliche Verbesserung gegenüber den bisher bekannten energieselektiven Detektoranordnungen auf.

Weitere vorteilhafte Ausführungen sind in den Unteransprüchen aufgezeigt.

Bei einer zeilenförmigen oder flächigen Anordnung derartiger Detektorpaare tritt der Effekt auf, daß sich Szintillationslicht im Detektormaterial ausbreitet und in benachbarten Photodioden nachgewiesen wird. Dieses sogenannte optische Übersprechen führt zu einer Kontrastminderung im Röntgenbild und verfälscht ebenfalls die Materialbestimmung. Dieser Effekt nimmt mit wachsender Materialdicke des Detektors und abnehmender Selbstabsorption des Szintillationslichts stark zu. Um diesem Effekt zu begegnen, wird der Detektor in einzelne Pixel segmentiert und durch eine Reflexionsschicht zum Beispiel aus Titanoxid oder Aluminiumoxid voneinander getrennt. Diese Trennung erfolgt sowohl beim Niederenergiedetektor als auch beim Hochenergiedetektor.

Anhand eines Ausführungsbeispiels mit Zeichnung soll die Erfindung näher erläutert werden. Es zeigt:
- Figur 1: eine Anordnung zur Detektion von Röntgenstrahlen mit zwei Detektoren
- Figur 2: eine Detektorzeile, bestehend aus Detektoren aus Figur 1, mit Auswerteeinheit und Monitor (leicht stilisiert)

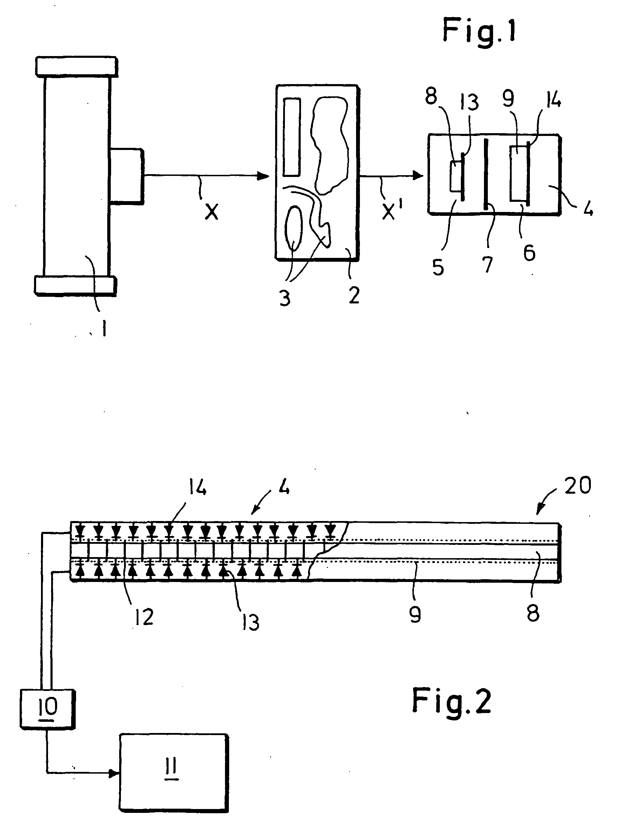

In Figur 1 ist ein Röntgenstrahlgenerator 1 dargestellt, in dem ein Röntgenstrahl X in einem Energiebereich von 10 bis 500 keV, vorzugsweise bis 140 keV, erzeugt und abgestrahlt wird. Dieser Röntgenstrahl X durchdringt ein im Strahlengang befindliches Objekt 2 mit verschiedenen Gegenständen 3, die aus unterschiedlichen Materialien bestehen können. Durch die unterschiedlichen Absorptionseigenschaften der einzelnen Materialien dieser Gegenstände 3 im Strahlengang gelangt ein in seiner spektralen Zusammensetzung veränderter und in seiner Intensität abgeschwächter Röntgenstrahl X' auf eine hier im Schnitt dargestellte Detektoranordnung 4.

Mehrere baugleiche Detektoren der Detektoranordnung 4 bilden vorzugsweise eine Detektorzeile 20, wie sie in Figur 2 dargestellt ist (wird noch ausgeführt). Die hier als Detektorpaar ausgebildete Einzeldetektoranordnung 4 setzt sich aus einem ersten vorderen Detektor 5 und einem zweiten hinteren Detektor 6 zusammen. Der erste Detektor 5 ist ein Niederenergiedetektor und absorbiert hauptsächlich den niederenergetischen Energiebereich bzw. Teil des abgeschwächten Röntgenstrahls X', während der zweite Detektor 6 ein Hochenergiedetektor ist und den hochenergetischen Bereich bzw. Teil des abgeschwächten Röntgenstrahls X' absorbiert. Entsprechend der Materialart und der Materialdicke des Materials der Gegenstände 3 trifft der Röntgenstrahl X' abgeschwächt auf die Detektoranordnung 4 (bzw. Detektorzeile 20) auf, in der entsprechende, die Absorptionseigenschaft der Materialien der Gegenstände 3 charakterisierende Signale in bekannter Art und Weise generiert werden. Diese werden als Einzelsignale an eine Auswerteeinheit 10 gegeben, ausgewertet und können zusätzlich als Bild auf einem Monitor 11 sichtbar gemacht werden (Figur 2).

Um bei der automatischen Materialartbestimmung eine genauere Aussage über das Material treffen zu können, ist es notwendig, die auszuwertenden Energien bzw. Energiebereiche voneinander zu trennen. Dies erfolgt hierbei durch die gezielte Wahl des Detektormaterials 8 des Niederenergiedetektors 5 unter Berücksichtigung des Detektormaterials 9 des Hochenergiedetektor 6.

Dieser Detektor 6 besteht vorzugsweise aus einer gepreßten Keramik, die mindestens ein Element mit einer Ordnungszahl größer als 60 enthält, wie beispielsweise Gadoliniumoxysulfid Gd₂ O ₂ S (SE), welches mit mindestens einem Selten-Erden-Element SE aus der aus Ce, Pr und Tb bestehenden Gruppe dotiert ist. Die Dicke des Detektormaterials 9 des Detektors 6 beträgt vorzugsweise 1,0 bis 2,0 mm. Auf dieses Detektormaterial 9 wird ein abgestimmtes Detektormaterial 8 für den Niederenergiedetektor 5 verwendet, der vorzugsweise aus einem Szintillationsmaterial, das mindestens ein Element mit einer Ordnungszahl von 30 bis 40 jedoch kein Element mit einer größeren Ordnungszahl enthält, besteht. Hierbei hat sich gezeigt, daß eine gute Auftrennung der Energiebereiche mit einem Detektormaterial 8 aus Zinkselenid mit Tellurdotierung ZnSe (Te) erreicht werden konnte. Dabei wurde dieses Zinkselenid in einer Dicke von 0,2 bis 1,0 mm verwendet. Weitere Vorteile dieser Materialkombination liegen in der Unempfindlichkeit gegenüber Feuchtigkeit und der an die spektrale Empfindlichkeit herkömmlicher Photodioden angepaßten Emissionswellenlängen des erzeugten Szintillationslichts.

Diese gezielte Wahl des Detektormaterials 8 für den Niederenergiedetektor 5 unter Berücksichtigung des Detektormaterials 9 für den Hochenergiedetektor 6 ermöglicht außerdem die gezielte Verwendung von aufeinander abgestimmten, gering nachleuchtenden Szintillations- bzw. Detektormaterialien 8, 9 sowohl für den Niederenergiedetektor 5 als auch für den Hochenergiedetektor 6. Insbesondere das Zinkselenid als auch das Gadoliniumoxysulfid besitzen jeweils eine kleine Nachleuchtdauer, die jeweils kleiner als der Zeitraum zwischen zwei Detektorauslesungen sind. Als Folge werden aussagekräftigere Meßergebnisse und konturenscharfe Darstellungen erreicht und angezeigt. Dazu wird in den Detektorschichten 8,9 die abgeschwächte Röntgenstrahlung (X') in sichtbare Strahlung umgewandelt und von den mit den Detektorschichten 8,9 korrespondierenden Halbleiterphotodioden 13,14 in ein Stromsignal umgesetzt, welches dann für die Auswertung an die Auswerteeinheit 10 einer nicht näher dargestellten Rechnereinheit eines Röntgenprüfgerätes bzw. Röntgenprüfsystems gegeben wird.
Bei einer zeilenförmigen oder flächigen Anordnung der Detektoranordnung 4 zu einer Detektorzeile 20 wird, um ein bekanntes optisches Übersprechen der nebeneinander angeordneten Detektorenanordnungen 4 zu vermeiden, das Detektor- bzw. Szintillationsmaterial 8, 9 in einzelne Pixel segmentiert. Die Trennung erfolgt durch eine Reflexionsschicht 12, die beispielsweise aus Titanoxid oder Aluminiumoxid besteht und zwischen den segmentierten Pixeln eingebracht ist.

Für eine weitere Auftrennung der Energien bzw. Energiebereiche des abgeschwächten Röntgenstrahls X' kann ein Filter 7, wie in Figur 1 erkennbar, zwischen den beiden Detektoren 5, 6 angeordnet werden. Dieses Filter 7 besteht vorzugsweise aus Kupfer und besitzt eine Dicke von 0,2 bis 1,0 mm, in dem noch im Röntgenstrahl X' nach Durchdringung des Detektors 5 verbliebene Niederenergieanteile absorbiert werden.

Diese Detektoranordnung 4 wird in einem hier nicht näher dargestellten Röntgengerät zur zerstörungsfreien Materialprüfung , zur Lebensmittelprüfung als auch zur Kontrolle von Gepäck und Frachtgut integriert.

Es versteht sich, daß im Rahmen der erfinderischen Idee Änderungen möglich sind. So ist die Detektoranordnung 4 nicht auf zwei Detektoren begrenzt.

## Patentansprüche

1. Verfahren zur zersförungsfreien Materialprifung, zur Lebensmittelprüfung oder zur Kontrolle von gepack und Fraditgut durch Ermittlung der Materialart der Gegenstände eines zu durchleuchtenden Objektes mit einer Detektoranordnung zur Detektion von Röntgenstrahlen,
bestehend aus wenigstens einem Niederenergiedetektor (5), dem wenigstens ein dahinter angeordneter Hochenergiedetektor(6) zugeordnet ist, wobei die einzelnen Energiebereiche des Röntgenstrahls zeitgleich auf ein zu durchleuchtendes Objekt (2) abgestrahlt und dabei abgeschwächt werden,
**dadurch gekennzeichnet,**
- **dass** der Niederenergiedetektor (5) als Detektormaterial (8) einen Szintillator aufinreist, der ein Einkristall ist,
- **dass** das Detektormaterial (8) des Niederenergiedetektors (5) Zinkselenid dotiert mit Tellur (ZnSe(Te)) ist,
- **dass** das Detektormaterial (9) des Hochenergiedetektors (6) keramisches Gadoliniumoxysulfid Gd₂O₂S (SE) dotiert mit mindestens einem Selten-Erden-Element (SE) aus der Ce, Pr und Tb bestehenden Gruppe ist,
- **dass** die Materialien des Niederenergiedetektors (5) und des Hochenergiedetektors (6) aufeinander abgestimmt sind und jeweils eine kleine Nachleuchtdauer aufweisen, die geringer sind als der Zeitraum zwischen zwei Detektorauslesungen, und
- **dass** das Detektormaterial (8) des Niederenergiedetektors (5) eine Dicke von 0,2 bis 1,0 mm aufweist.

2. Verfahren nach Anspruch 1, **dadurch gekennzeichnet, daß** das Detektormaterial (9) des Hochenergiedetektors (6) eine Dicke von 1,0 bis 2,0 mm besitzt.

3. Verfahren nach einem der Ansprüche 1 oder 2, **dadurch gekennzeichnet, daß** der Niederenergiedetektor (5) oder der Hochenergiedetektor (6) oder beide durch Reflexionsschichten (12) in einzelne Segmente separiert werden.

4. Verfahren nach Anspruch 3, **dadurch gekennzeichnet, daß** die Reflexionsschicht (12) ein Titanoxid ist.

5. Verfahren nach Anspruch 3, **dadurch gekennzeichnet, daß** die Reflexionsschicht (12) ein Aluminiumoxid ist.

6. Verfahren nach einem oder mehreren der vorgenannten Ansprüche, **dadurch gekennzeichnet, daß** das Detektormaterial (8,9) mit zugeordneten Halbleiterphotodioden (13,14) korrespondiert, wobei die abgeschwächte Röntgenstrahlung (X') in eine sichtbare Strahlung und danach in ein elektrisches Stromsignal umgewandelt wird.

7. Verfahren nach einem oder mehreren der vorgenannten Ansprüche, **dadurch gekennzeichnet, daß** zwischen dem Niederenergiedetektor (5) und dem Hochenergiedetektor (6) ein Filter (7) eingebracht ist.

## Claims

1. Method for destruction-free material testing, for food testing or for monitoring baggage and freight by ascertaining the material type of an object to be transilluminated with a detector arrangement for detecting X-rays, consisting of at least one low-energy detector (5), to which at least one high-energy detector (6) arranged downstream thereof is assigned, wherein the individual energy ranges of the X-ray are transmitted simultaneously onto an object (2) to be transilluminated and are attenuated in the process, **characterized in that**
- the low-energy detector (5) has as detector material (8) a scintillator which is a single crystal,
- the detector material (8) of the low-energy detector (5) is zinc selenide doped with tellurium (2nSe(Te)),
- the detector material (9) of the high-energy detector (6) is ceramic gadolinium oxysulfide Gd₂O₂S (SE) doped with at least one rare-earth element (SE) from the group consisting of Ce, Pr and Tb,
- the materials of the low-energy detector (5) and of the high-energy detector (6) are matched to one another and in each case have a small afterglow duration, which afterglow durations are less than the interval between two detector readings, and
- the detector material (8) of the low-energy detector (5) has a thickness of 0.2 to 1.0 mm.

2. Method according to Claim 1, **characterized in that** the detector material (9) of the high-energy detector (6) has a thickness of 1.0 to 2.0 mm.

3. Method according to one of Claims 1 or 2, **characterized in that** the low-energy detector (5) or the high-energy detector (6) or both are separated into individual segments by reflection layers (12).

4. Method according to Claim 3, **characterized in that** the reflection layer (12) is a titanium oxide.

5. Method according to Claim 3, **characterized in that** the reflection layer (12) is an aluminium oxide.

6. Method according to one or more of the preceding claims, **characterized in that** the detector material (8, 9) corresponds to assigned semiconductor photodiodes (13, 14), wherein the attenuated X-radiation (X') is converted into visible radiation and subsequently into an electrical current signal.

7. Method according to one or more of the preceding claims, **characterized in that** a filter (7) is introduced between the low-energy detector (5) and the high-energy detector (6).

## Revendications

1. Procédé de contrôle non destructif de matériau, de contrôle de denrée alimentaire ou de contrôle de bagages et de marchandises par détermination du type de matériau des articles d'un objet à radiographier avec un arrangement de détecteurs destinés à détecter des rayons X, composé d'au moins un détecteur à basse énergie (5) auquel est associé au moins un détecteur à haute énergie (6) disposé derrière lui, les plages énergétiques individuelles des rayons X étant émises de manière isochrone sur un objet à radiographier (2) et étant ainsi affaiblies,
**caractérisé en ce**
- **que** le détecteur à basse énergie (5) présente comme matériau de détecteur (8) un scintillateur qui est un monocristal,
- **que** le matériau de détecteur (8) du détecteur à basse énergie (5) est du séléniure de zinc dopé avec du tellure (2nSe(Te)),
- **que** le matériau de détecteur (9) du détecteur à haute énergie (6) est un sulfure d'oxyde de gadolinium céramique Gd₂O₂S(SE) dopé avec au moins un élément de terre rare (SE) du groupe composé de Ce, Pr et Tb,
- **que** les matériaux du détecteur à basse énergie (5) et du détecteur à haute énergie (6) sont accordés l'un à l'autre et présentent respectivement un petit temps de persistance de luminescence, lequel est inférieur à la durée entre deux lectures du détecteur, et
- **que** le matériau de détecteur (8) du détecteur à basse énergie (5) présente une épaisseur de 0,2 à 1,0 mm.

2. Procédé selon la revendication 1, **caractérisé en ce que** le matériau de détecteur (9) du détecteur à haute énergie (6) possède une épaisseur de 1,0 à 2,0 mm.

3. Procédé selon l'une des revendications 1 ou 2, **caractérisé en ce que** le détecteur à basse énergie (5) ou le détecteur à haute énergie (6) ou les deux sont divisés en segments individuels par des couches réfléchissantes (12).

4. Procédé selon la revendication 3, **caractérisé en ce que** la couche réfléchissante (12) est un oxyde de titane.

5. Procédé selon la revendication 3, **caractérisé en ce que** la couche réfléchissante (12) est un oxyde d'aluminium.

6. Procédé selon l'une ou plusieurs des revendications précédentes, **caractérisé en ce que** le matériau de détecteur (8, 9) correspond à des photodiodes semiconductrices (13, 14) associées, les rayons X (X') affaiblis étant convertis en un rayonnement visible et ensuite en un signal de courant électrique.

7. Procédé selon l'une ou plusieurs des revendications précédentes, **caractérisé en ce qu'**un filtre (7) est monté entre le détecteur à basse énergie (5) et le détecteur à haute énergie (6).
